# EUROPEAN PATENT SPECIFICATION

(11) **EP 2 579 858 B1**
(45) Date of publication and mention of the grant of the patent: **21.05.2014**
(21) Application number: 11727948.9
(22) Date of filing: 14.06.2011
(51) Int. Cl.: A61K 9/20, A61K 31/55, A61K 9/14, A61K 9/16, C07D 233/16

(54) **IVABRADINE-CONTAINING PHARMACEUTICAL COMPOSITION**
IVABRADINHALTIGE PHARMAZEUTISCHE ZUSAMMENSETZUNG
COMPOSITION PHARMACEUTIQUE CONTENANT DE L'IVABRADINE

(30) Priority: 23.06.2010 IN CH17602010; 14.06.2010 EP 10165884; 14.06.2010 EP 10165881
(43) Date of publication of application: 17.04.2013
(73) Proprietor: Ratiopharm GmbH, 89079 Ulm (DE)
(72) Inventor: MEERGANS, Dominique, 81477 München (DE); RIMKUS, Katrin, 82049 Pullach (DE); GEIER, Jens, 89610 Oberdischingen (DE)
(74) Representative: Teipel, Stephan
(86) International application number: PCT/EP2011/059865
(87) International publication number: WO 2011/157721

(56) References cited:
- WO-A1-2009/124940
- US-A1- 2005 106 238
- US-A1- 2007 135 411
- Bauer, K.H., Frömming, K.-H., Führer, C.: "Lehrbuch der Pharmazeutischen Technologie", 1999, Wissenschaftliche Verlagsgesellschaft mbH Stuttgart, Stuttgart, Deutschland, XP002605588, ISBN: 3-8047-1700-4 page 133, Absatz 3.1.3 "Entmischungen"

## Description

The present invention relates to a pharmaceutical composition containing ivabradine adipate. Further, the invention relates to a method for the preparation of such a composition.

Ivabradine has the chemical designation (S)-3-{3-[(3,4-dimethoxybicyclo[4.2.0]octa-1,3,5-triene-7-ylmethyl)methylamino]propyl}-7,8-dimethoxy-2,3,4,5-tetrahydro-1H-3-benzazepine-2-one. Ivabradine has the following structural formula (I):

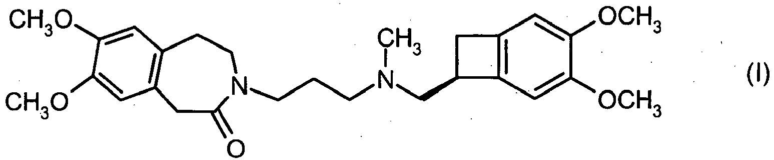

Synthesis routes for the preparation of ivabradine and its use for preventing and treating various clinical conditions of myocardial ischaemia, supraventricular arrhythmias and coronary arteriosclerotic episodes are reported to be disclosed in EP 534 859.

Ivabradine is an active substance reported to have a bradycardic effect for the treatment of stable angina pectoris, in particular in patients for whom beta-blockers are contraindicated or an intolerance of beta-blockers is present. Ivabradine is reported to selectively inhibit the I_{f}-ion current, which, as an intrinsic pacemaker in the heart, controls the spontaneous diastolic depolarisation in the sino-atrial node and thus regulates the heart rate. Under physiological conditions, ivabradine, the S-enantiomer of a racemate, is reported to have a very good solubility (> 10 mg/ml).

The prior art apparently discloses administration forms of ivabradine, which release the active substance substantially without a time delay. The administration form Procoralan^{®} (Servier), which is prepared by wet granulation, releases ivabradine rapidly and almost completely after oral intake. WO 2003-061662 apparently discloses an ivabradine-containing, orally dispersible tablet, which releases the active substance very rapidly in the mouth.

Moreover, various polymorphic forms of the ivabradine hydrochloride are reported to be described in the state of the art. WO 2005/110993 A1 apparently discloses polymorph alpha, WO 2006/092493 A1 apparently discloses polymorph beta, WO 2006/092491 A1 apparently discloses polymorph beta d (dehydrated). In addition, polymorph gamma, polymorph gamma d, polymorph delta, and polymorph delta d are reported to be known in the art. In addition, WO2008/065681 apparently reports the so-called Form I of Ivabradine HCl. WO 2008/146308 A2 apparently discloses amorphous ivabradine.

Also various salts of ivabradine are apparently known in the art. WO 2008/146308 A2 apparently discloses ivabradine oxalate, WO 2009/124940 A1 discloses ivabradine hydrobromide.

The problem with the salts and polymorphs of the ivabradine, in particular the polymorphs of the hydrochloride, is that these salt forms are not sufficiently stable under all conditions. This, in turn can lead to problems in the processing as well as the storage and to undesired reactions with the excipients employed in the preparation of the pharmaceutical composition.

Thus, it is an object of the present invention to provide a pharmaceutical composition in the preparation and later storage of which the employed polymorphic form of the active substance is stable.

A further problem with the ivabradine-containing pharmaceutical compositions is that the amount of active substance in the formulation to be administered is usually only small. This leads to problems in the preparation of the corresponding compositions due to possible variations in content that are for example conditional on separation tendencies of the active substances and excipients. Therefore, it is important that at first active substances and excipients can be mixed as homogenous as possible and corresponding mixtures do not separate again during further processing to the final formulation. An inhomogeneous distribution of the active substance can result in undesired side effects up to symptoms of poisoning. Also the bioavailability as well as the effectiveness of corresponding formulations may be affected adversely in an inhomogeneous distribution of the active substance.

It has been shown that neither problems regarding the stability of the employed polymorphic form of the active substance nor problems regarding the homogeneous distribution of the active substance in the final formulation can be solved by simply mixing and compressing the constituents.

Thus, a further object of the present invention is to provide a pharmaceutical composition that ensures a distribution of the active substance in the final formulation that is as homogeneous as possible. At the same time, the employed polymorphic form should remain stable both in the preparation of the composition and the later storage.

Now, it has surprisingly been found that the above-mentioned problems can be solved by the subject matter as claimed.

Thus, the present invention relates to a pharmaceutical composition containing ivabradine adipate as active substance wherein the active substance has an average particle size in the range of 0.5 µm to 250 µm.

Presently, by "active substance" ivabradine adipate is meant. The salt of the ivabradine can be obtained in accordance to methods reported to be known in the art by reacting the free base of the ivabradine with the corresponding acid or by the presence of the corresponding acid in the synthesis of the ivabradine, as reported to be described for example in US 2005/0228177 A1.

In particular, if ivabradine is used as adipate salt, the pharmaceutical composition according to the present invention is stable under usual storage conditions.

The active substance can be present in the pharmaceutical composition of the present invention both in the crystalline and amorphous form. The active substance includes all polymorphic forms of ivabradine or a pharmaceutically acceptable salt thereof, including hydrates and solvates. Preferably, the active substance is present in the crystalline form.

Ivabradine adipate can be obtained by adding adipic acid, e.g. about one equivalent, in a suitable solvent, such as ethanol, to a solution of ivabradine in a suitable solvent, such as dichlormethane. Crystalline ivabradine adipate product can be obtained by removal of the solvent, e.g. under vacuum at about 40°C. Crystalline ivabradine adipate can also be obtained by adding a solution of adipic acid in water to a solution of ivabradine in ethanol, and removal of the solvent.

The DSC thermogramm of ivabradine adipate shows a peak at about 115°C. The melting point is in the range of about 113°C to about 117°C.

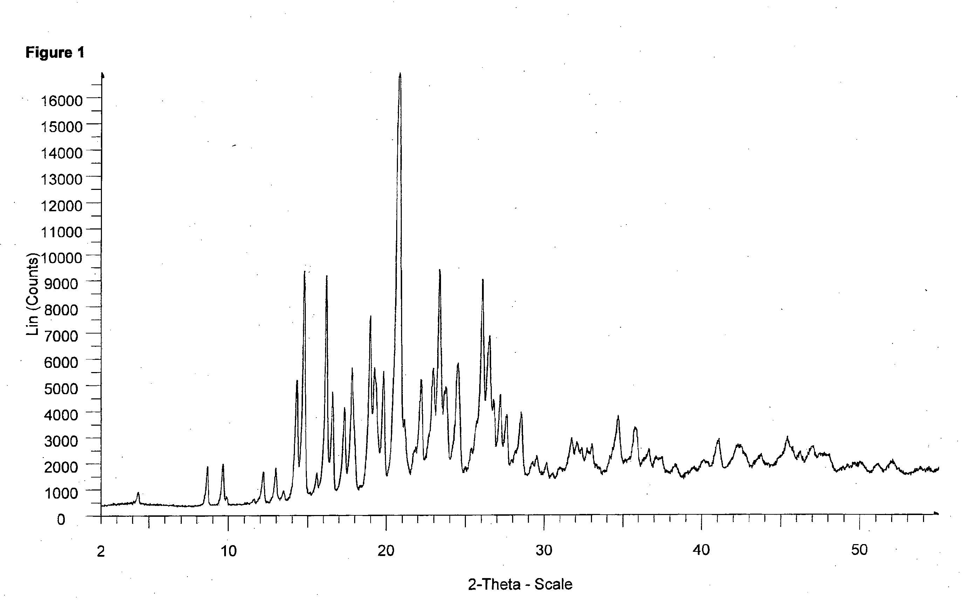

Ivabradine adipate is characterized by an XRD pattern having a characteristic peak at 20.6 ± 0.2 degrees 2-theta, in particular having characteristic peaks at 14.6 ± 0.2, 16.0 ± 0.2, 18.8 ± 0.2, 20.6 ± 0.2, 23.2 ± 0.2, 24.3 ± 0.2, 25.9 ± 0.2 and 26.3 ± 0.2 degrees 2-theta, and further at 8.6 ± 0.2, 9.6 ± 0.2, 12.1 ± 0.2 and 12.9 ± 0.2 degrees 2-theta. The XRD pattern of ivabradine adipate is shown in Figure 1.

It has been shown that the uniformity of the content of active substance of ivabradine-containing pharmaceutical compositions can be ensured when the average particle size of the active substance is in the range of 0.5 µm to 250 µm. This way, the separation tendency in the preparation of the composition is reduced so that the variations in content in the finished composition can be prevented. Moreover, it has surprisingly shown that the pharmaceutical composition can be prepared by simple mixing and compressing with correspondingly small active substance particles without leading to a change of otherwise instable polymorphic forms of the active substance. This way it is possible to obtain the pharmaceutical composition according to the invention without the necessity of an otherwise usual and for the commercial ivabradine-containing drug Procoralan^{®} used wet granulation by a simple dry processing of the constituents. So, the employment of special machines necessary for the wet granulation can be avoided. Moreover, the employment of solvents for the preparation of the wet mass can be avoided. It is also not necessary to expose the active substance for a longer period to the granulation liquid until the completion of the drying. In addition, the drying step following the wet granulation requires additional energy and the active substance is exposed to thermal influences over a longer period. In contrast, using the active substance with a particle size in the range of 0.5 µm to 250 µm permits the preparation of the pharmaceutical composition according to the invention by direct compressing or dry compaction in the absence of solvents so that the above-mentioned problems in the preparation of conventional ivabradine-containing formulations are overcome. The preparation of the pharmaceutical composition according to the invention by direct compressing is particularly preferred.

The pharmaceutical composition according to the invention contains the active substance in an average particle size in the range of 0.5 µm to 250 µm, preferably in the range of 0.8 µm to 200 µm, in particular in the range of 1 µm to 150 µm.

In a further embodiment of the present invention the pharmaceutical composition contains particles of the active substance having an average particle size D50 in the range of 1 µm to 70 µm, preferably of 5 µm to 50 µm, most preferably of 10 µm to 25 µm.

In a further embodiment of the present invention the pharmaceutical composition contains particles of the active substance having an average particle size D90 in the range of 0.5 µm to 250 µm, preferably of 30 µm to 80 µm, most preferably of 40 µm to 60 µm.

The term "particle size" according to the present invention relates to the maximum diameter of the equivalent product assuming spherical opaque particles showing the same light scattering pattern and the same diffraction as the active substance particles. According to the invention the particle size is determined by means of laser light diffraction. The determination of the size distribution results from the analysis of the diffraction pattern that is obtained if particles are exposed to a monochromatic light beam. The particles refract the light with small particles refracting the light in a greater angle than large particles. The refracted light is measured by a number of photo detectors arranged in different angles. On the other hand, the light spectra of the small particles have to be recorded by light-sensitive detectors in greater angles over the laser beam. Large particles result in greater intensity maxima with small angles, small particles to weaker intensity maxima with greater angles. Thus, in the laser light diffraction the pattern resulting from the interaction of the light with the particles is used for the determination of the particle size.

The "particle size distribution" is a statistical frequency distribution. Here, the particles are divided into classes according to their size.

The particle size distribution of the particle size D50 value includes 50% of the particles based on their volume with a particle size smaller than the D50 value and 50% of the particles based on their volume with a particle size greater than the D50 value.

The particle size distribution of the particle size D90 value includes 90% of the particles based on their volume with a particle size smaller than the D90 value and 90% of the particles based on their volume with a particle size greater than the D90 value.

The particle size distribution according to the present invention can be monomodal or bimodal. In the preferred embodiment of the invention the particle size distribution of the active substance is monomodal. The term "monomodal" relates to the peak resulting in a histogram and/or graph representing the distribution frequency. Generally, in the graphical representation of a particle size distribution there are plotted the diameter x on the abscissa and the measure of a set Q on the ordinate.

According to the invention the particle size is determined by means of laser diffractometry. For that, a Mastersizer 2000 by Malvern Instruments having the corresponding sample dispersing unit Hydro S is used. The wet measurement (2500 rpm, ultrasound 10-20 min., shading 5 to 20%) takes place in a dispersion of sunflower oil with the particle spacing in the dispersion being about 3-5 times greater than the particle diameter.

Here, the average particle size of the active substance is determined according to the following method: In principle, the Fraunhofer diffraction theory is used for particle fractions the particle size of which is significantly greater than the wave length of the laser light. (ISO 13320)

Moreover, the Mie theory defines the secondary scattering caused by the refraction of the light on small particles, as in the international rules of the laser diffraction measurement. (ISO 13320)

The determination of the particle size for particles D50 smaller than 5.0 µm is carried out according to the Mie method and for particles D50 greater than 5.0 µm according to the Fraunhofer method.

In a further aspect of the present invention it has been shown that the separation tendency of ready-made mixtures containing the active substance and the excipients is reduced in the further processing by addition of an adhesion enhancer. Additionally, it has been shown that an adhesion enhancer is suitable to stabilize the polymorphic form of the employed active substance in compacted or compressed form. By adding the adhesion enhancer it usually comes to an enlargement of the interparticle surfaces at which more easily (e.g. in the compressing operation) contact points can be formed. Moreover, adhesion enhancers are wherein they increase the plasticity of the tabletting mixture so as to form solid tablets during compressing.

Particularly suitable as adhesion enhancers are polymers, fats, waxes, non-polymeric compounds having at least one polar side group. The employed adhesion enhancer should be in the solid form at room temperature.

In one embodiment of the present invention the employed adhesion enhancer is a polymer that has a glass transition temperature (Tg) of >15°C, preferably 40°C to 150°C, and in particular 50°C to 100°C. Here, the glass transition temperature is that temperature at which the amorphous or partly crystalline polymer changes from the solid to the liquid state. Here, a significant change of physical parameters such as hardness and elasticity occurs. Typically, below the glass transition temperature a polymer is glassy and hard, above the glass transition temperature it changes into a rubber-like to viscous state. The determination of the glass transition temperature takes place in the context of this invention by means of differential scanning calorimetry (DSC). For that, for example a device of Mettler Toledo DSC 1 can be used. It works with a heating rate of 10°C.

The polymer used as the adhesion enhancer preferably has a number average molecular weight of 1,000 g/mol to 500,000 g/mol, more preferred of 2,000 g/mol to 90,000 g/mol. Additionally, the polymer used should have a viscosity of 0.1 mPa/s to 8 mPa/s, preferably of 0.3 mPa/s to 7 mPa/s, and in particular of 0.5 mPa/s to 4 mPa/s in a 2% by weight solution in water, each measured at 25°C.

Preferably, there can be employed hydrophilic polymers as the adhesion enhancers. This refers to polymers having hydrophilic groups, for example hydroxy, alkoxy, acrylate, methacrylate, sulfonate, carboxylate, and quarternary ammonium groups.

According to the invention the polymer used as the adhesion enhancer can be selected from the group consisting of polysacharides, such as hydroxypropylmethylcellulose (HPMC), carboxymethylcellulose (CMC), ethylcellulose, methylcellulose, hydroxyethylcellulose, ethylhydroxyethylcellulose, and hydroxypropylcellulose (HPC), micro-crystalline cellulose, guar gum, alginic acid, alginates, polyvinylpyrrolidone, polyvinylacetates (PVAC), polyvinyl alcohols (PVA), polymers of the acrylic acid and its salts, polyacrylamides, polymethacrylates, vinylpyrrolidone vinylacetate copolymers, polyalkylene glycoles, such as poly(propylene glycol) and polyethylene glycol, co-blockpolymers of the polyethylene glycol, in particular co-blockpolymers of polyethylene glycol and poly(propylene glycol) as well as mixtures of two or more of the mentioned polymers.

Preferably used as the adhesion enhancers are polyvinylpyrrolidone, especially having a weight average molecular weight of 10,000 g/mol to 60,000 g/mol, in particular 12,000 g/mol to 40,000 g/mol, copolymers from vinylpyrrolidone and vinylacetate, in particular having a weight average molecular weight of 40,000 g/mol to 70,000 g/mol, polyethylene glycol, in particular having a weight average molecular weight of 2,000 g/mol to 10,000 g/mol, as well as HPMC, in particular having a weight average molecular weight of 20,000 g/mol to 90,000 g/mol and/or a proportion of methyl groups of 10% to 35% and/or a proportion of hydroxy groups of 1% to 35%. Further, microcrystalline cellulose can be used, in particular those having a specific surface area of 0.7 m²/g to 1.4 m²/g. The determination of the specific surface area takes place by means of the gas adsorption method in accordance to Brunauer, Emmet and Teller.

Suitable non-polymeric compounds having at least one polar side group are in particular sugar alcohols and disaccharides, wherein the term sugar alcohols in this case also comprises monosaccharides. Examples of suitable sugar alcohols/disaccharides are lactose, mannitol, sorbitol, xylitol, isomalt, glucose, fructose, maltose, and mixtures of two or more of these compounds.

Alternatively, also waxes such as for example hexadecyl palmitate or carnauba wax can be used as adhesion enhancers. Also, fats such as glycerol fatty acid esters (e.g., glycerolpalmitate, glycerolbehenate, glycerollaurate, and glycerolstearate) or PEG glycerol fatty acid esters can be used.

All of the above-mentioned adhesion enhancers can be employed alone or as a mixture of two or more of the mentioned compounds.

It is advantageous if the adhesion enhancer is used in the particulate form and has a volume average particle size of 5 µm to 200 µm.

The weight ratio of the active substance to the adhesion enhancer in the pharmaceutical composition according to the invention can be freely selected by the skilled person depending on the active substance used and the adhesion enhancer as well as the desired composition. Preferably, the weight ratio of ivabradine based on the free base to adhesion enhancer is in the range of 10:1 to 1:100, more preferred in the range of 1:1 to 1:75, more preferred in the range of 1:2 to 1:50, and most preferred in the range of 1:5 to 1:35.

For example, the pharmaceutical composition of the present invention can contain 1-80% by weight, more preferred 2-60% by weight, in particular 2-40% by weight, and especially 3-5% by weight ivabradine adipate, based on the free base of the active substance and the total weight of the composition. Here and in the following, by total weight of the composition the weight of the composition without optionally present film coatings is to be understood.

Additionally, the pharmaceutical composition can contain one or more further pharmaceutically acceptable excipients, such as e.g. fillers, glidants, flow regulators, release agents, and disintegrants. ("Lexikon der Hilfsstoffe für Pharmazie, Kosmetik und angrenzende Gebiete", edited by H. P. Fiedler, 4th Edition, and "Handbook of Pharmaceutical Excipients", 3rd Edition, edited by Arthur H. Kibbe, American Pharmaceutical Association, Washington, USA, and Pharmaceutical Press, London).

Fillers: The pharmaceutical composition can contain one or more filler(s). In general, a filler is a substance that increases the bulk volume of the mixture and thus the size of the resulting pharmaceutical dosage form. Preferred examples of fillers are lactose and calcium hydrogenphosphate. The filler may be present in a proportion of 0 to 80% by weight, preferred between 10 and 60% by weight of the total weight of the composition.

Glidants: The function of the glidant is to ensure that the pelletizing and the ejection take place without much friction between the solids and the walls. Preferably, the glidant is an alkaline earth metal stearate, e.g. magnesium stearate, or a fatty acid, such as stearic acid. Typically, the glidant is present in an amount of 0 to 2% by weight, preferably between 0.5 and 1.5% by weight of the total weight of the pharmaceutical composition.

Disintegrants: Usually, by a disintegrant is meant a substance that is capable of breaking up the tablet into smaller pieces as soon as it is in contact with a liquid. Preferred disintegrants are croscarmellose sodium, sodium carboxymethyl starch, cross-linked polyvinylpyrrolidone (crospovidon), sodium carboxymethyl glycolate (e.g. explotab) and sodium bicarbonate. Typically, the disintegrant is present in an amount of 0 to 20% by weight, preferably between 1 and 15% by weight of the total weight of the composition.

Flow regulators: As the flow regulator there can be used e.g. colloidal silica. Preferably the flow regulator is present in an amount of 0 to 8% by weight, more preferably in an amount between 0.1 and 3% by weight of the total weight of the composition.

Release agents: The release agent can be e.g. talcum and is present in an amount between 0 and 5% by weight, preferably in an amount between 0.5 and 3% by the weight of the composition.

Normally, the pharmaceutical composition according to the invention has a uniformity of the active substance content (content uniformity) of 85% to 115%, preferably 90% to 110%, in particular 95% to 105% of the average content. That is, all dosage forms, for example tablets, have a content of active substance between 85% and 115%, preferably between 90% and 110%, in particular between 95% and 105% of the average active substance content. The "content uniformity" is determined according to Ph. Eur. 6.0, section 2.9.6.

The pharmaceutical composition of the present invention may be for example in the form of tablets, granules, or pellets. Here, the granule or the pellets for example may be present in capsules or sachets. Preferred are tablets that may have a film coating.

In a further preferred embodiment the pharmaceutical composition of the present invention is obtainable by dry granulation methods or direct compression methods in the absence of solvents.

Moreover, the present invention relates to a method for the preparation of a pharmaceutical composition as described above wherein the method comprises the steps:
a) obtaining ivabradine adipate wherein the active substance has an average particle size in the range of 0.5 µm to 250 µm, preferably of 0.8 µm to 200 µm, most preferably of 1 µm to 150 µm; and
b) mixing the active substance with one or more pharmaceutically acceptable excipients.

In the above step a) the active substance is obtained in the mentioned average particle size. This can be done in that the active substance is either provided with the desired particle size or an active substance having a greater particle size is at first transferred to particles of a smaller particle size, for example by grinding and/or screening.

In a preferred embodiment of the method according to the invention as an additional step there is admixed an adhesion enhancer. Suitable adhesion enhancers are the above-mentioned compounds. When an adhesion enhancer is admixed, it is preferred that at least a part of the adhesion enhancer, preferably the complete adhesion enhancer, is (pre-)mixed with the active substance some time, preferably about 5 to about 30 min., more preferably about 5 to about 10 min., e.g. about 10 min., before subjecting the mixture and optionally further excipients, to further process steps, e.g. dry granulation or direct compression, preferably direct compression. It has been surprisingly found that premixing the active substance and at least part of the adhesion enhancer followed by a short time delay advantageously influences the dissolution profile of the obtained composition, in particular of tablets.

Finally, the method according to the invention in a further preferred embodiment comprises the additional step of dry granulation or direct compressing in the absence of solvents, preferably direct compression. Doing so, there may be obtained for example tablets, which if desired subsequently can be provided with a film coating.

Preferably, the pharmaceutical composition according to the invention is present as a tablet containing ivabradine adipate in an amount preferably of 1 mg to 20 mg, more preferred 3 mg to 15 mg, in particular 5 mg to 10 mg, based on ivabradine free base. Thus, object of the invention are in particular tablets containing 5 mg or 7.5 mg ivabradine adipate, based on ivabradine free base.

Preferably, the pharmaceutical composition according to the invention is administered twice a day.

In a preferred embodiment, the oral administration of the formulation according to the invention to a human as a patient leads to a plasma level profile which is distinguished by a cₘₐₓ (maximum plasma level) based on a twice daily intake of 5 mg of the active substance ivabradine, in the steady state, of about 5 to 40 ng/ml, preferably 10 to 30 ng/ml.

The abovementioned values for the plasma level are preferably mean values, obtained by investigations of blood samples of a group of 10 test subjects (having an average body weight of 70 kg), the corresponding blood samples having been taken 0, 1, 2, 4, 6, 8, 12, 24 and 48 hours after oral administration of the composition according to the invention in the steady state. The determination of the plasma level values can preferably be carried out by suitable HPLC-MSMS methods.

Attached Figure 1 shows an XRD pattern of ivabradine adipate.

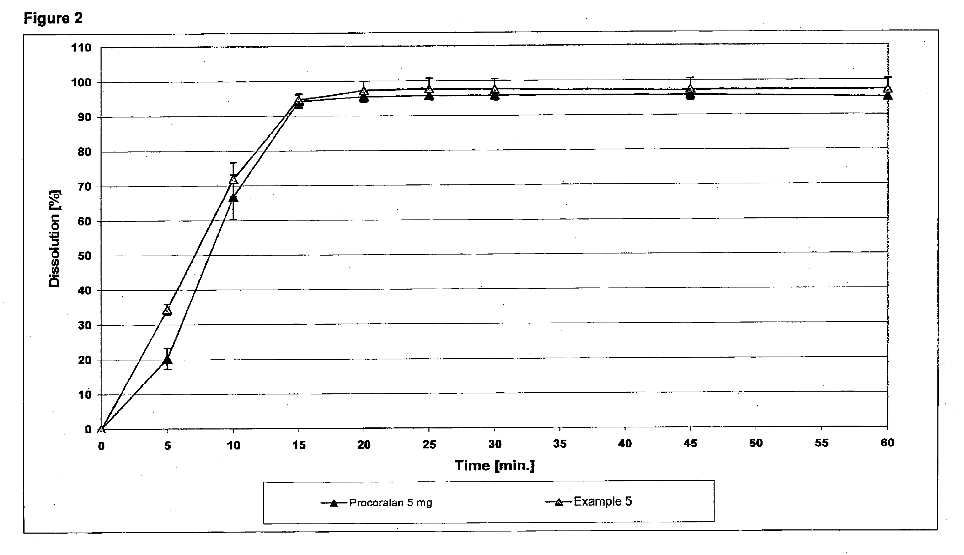

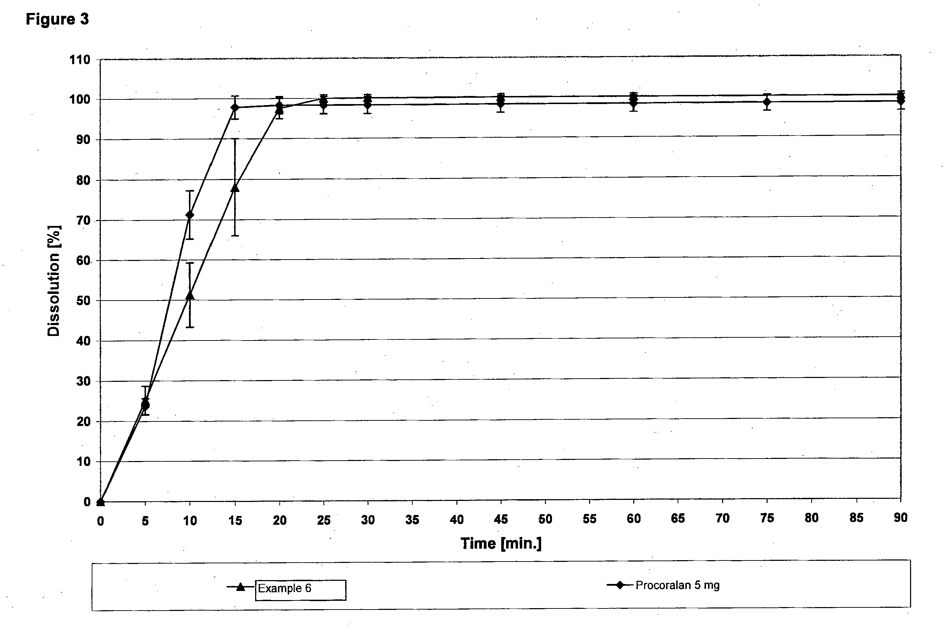

Figures 2 and 3 show dissolution profiles of the compositions of examples 5 and 6, respectively.

XRD samples were analysed on a Bruker-AXS D8 Advance powder X-Ray diffractometer. The measurement conditions were as follow :
Measurement in Bragg-Brentano-Geometry on vertical goniometer (reflection, theta/theta, 435 mm measurement circle diameter) with sample rotation (30 rpm) on 9 position sample stage
   - Radiation:: Cu Kα1(1.5406Å), Tube (Siemens FLCu2K), power 40kV/40mA
   - Detector:: position sensitive detector VANTEC-1
   3° capture angle (2theta),
   Anti scatter slit 6.17 mm
   Detector slit 10.39 mm
   4° soller slit,
   primary beam stop (<2° 2theta)
   - Monochromator:: None
   - Second β filter:: Ni filter 0.1 mm (0.5%)
   - Start angle:: 2°
   - End Angle:: 55°
   - Measurement time:: 11 min
   - Step:: 0.016° 2Theta
   - Software:: EVA (Bruker-AXS, Karlsruhe).

Now, the present invention is explained in more detail with respect to the following examples without these should be interpreted as being restrictive.

### Example 1: Direct compression

- Ivabradine adipate: 6.51 mg
- Avicel PH101: 50.00 mg
- Calcium hydrogenate phosphate: 25.00 mg
- Sodium croscarmelose: 14.91 mg
- Aerosil: 2.58 mg
- Magnesium Stearate: 1.00 mg

Ivabradine adipate together with Avicel PH101 was sieved through a 355 µm sieve and pre-mixed for 10 minutes in the tumbling mixer (Turbula T10B). Subsequently, all the other constituents except for magnesium stearate were added through the 355 µm sieve and stirred for further 30 minutes in the tumbling mixer. After the addition of magnesium stearate it was stirred again for 2 minutes in the tumbling mixer. The finished mixture was compressed on a rotary press (Riva Piccola) with 7 mm round biconvex punch. The tablets had a hardness ofabout 50-85 N.

### Example 2: Direct compression

- Ivabradine adipate: 6.51 mg
- Povidon VA 64: 10.00 mg
- Prosolv SMCC 90: 64.00 mg
- Sodium Bicarbonate: 14.91 mg
- Talcum: 1.00 mg
- Aerosil: 2.58 mg
- Magnesium Stearate: 1.00 mg

Ivabradine adipate together with Povidon VA 64 and Prosolv SMCC 90 was sieved through a 355 µm sieve and pre-mixed for 10 min. in the tumbling mixer (Turbula T10B). Subsequently, all the other constituents except for magnesium stearate were added through the 355 µm sieve, and stirred for further 30 min. in the tumbling mixer. After the addition of magnesium stearate it was stirred again for 2 min. in the tumbling mixer. The finished mixture was compressed on a rotary press (Riva Piccola) with 7 mm round biconvex punch. The tablets had a hardness of about 50-85 N.

### Example 3: Dry compacting (mixture corresponding to Example 2)

- Ivabradine adipate: 6.51 mg
- Povidon VA 64: 10.00 mg
- Prosolv SMCC 90: 64.00 mg
- Sodium Bicarbonate: 14.91 mg
- Talcum: 1.00 mg
- Aerosil: 2.58 mg
- Magnesium Stearate: 1.00 mg

Ivabradine adipate together with Povidon VA 64 and half of the Prosolv SMCC 90, magnesium stearate, Aerosil and the total amount of sodium bicarbonate were pre-mixed for 5 min. in the tumbling mixer (Turbula T10B) and compacted. Subsequently, the material was broken over a 1000 pm screen-type mill (Comil), the remaining excipients were added and the composition was mixed for 5 min. in the tumbling mixer. The finished mixture was compressed on a rotary press (Riva Piccola) with 7 mm round biconvex punch. The tablets had a hardness of about 50-85 N.

### Example 4: Direct compression (not according to the invention)

- Ivabradine HCl form I: 5.42 mg
- Avicel PH101: 50.00 mg
- Calcium hydrogen phosphate: 26.09 mg
- Sodium croscarmelose: 14.91 mg
- Aerosil: 2.58 mg
- Magnesium stearate: 1.00 mg

Ivabradine together with Avicel PH101 was pre-mixed for 10 min. in the tumbling mixer (Turbula T10B). Subsequently, all other constituents except for magnesium stearate were added, and stirred for further 30 min. in the tumbling mixer. After the addition of magnesium stearate it was stirred again for 2 min. in the tumbling mixer. The finished mixture was compressed on a rotary press (Riva Piccola) with 7 mm round biconvex punch. The tablets had a hardness of about 50-85 N.

### Example 5: Direct compression (not according to the invention)

- Ivabradine HCl form I: 5.42 mg
- Povidon VA 64: 11.09 mg
- Prosolv SMCC 90: 64.00 mg
- Sodium bicarbonate: 14.91 mg
- Talcum: 1.00 mg
- Aerosil: 2.58 mg
- Magnesum stearate: 1.00 mg

Ivabradine together with Povidon VA 64 and Prosolv SMCC 90 was sieved through a 355 µm sieve and pre-mixed for 10 min. in the tumbling mixer (Turbula T10B). Subsequently, all other constituents except for magnesium stearate were added through the 355 µm sieve and stirred for further 30 min. in the tumbling mixer. After the addition of magnesium stearate it was stirred again for 2 min. in the tumbling mixer. The finished mixture was compressed on a rotary press (Riva Piccola) with 7 mm round biconvex punch. The tablets had a hardness of about 50-85 N.

The dissolution profile (conditions: 500 mL 0.1 nHCl pH 1.2, 37°C, 50 rpm baskets (USP app. I)) of the tablets of Example 5 is shown in Fig. 2.

### Example 6: Dry compacting (mixture according to Example 5, not according to the invention)

- Ivabradine HCl form I: 5.42 mg
- Povidon VA 64: 10.00 mg
- Prosolv SMCC 90: 64.00 mg
- Sodium bicarbonate: 15.00 mg
- Talcum: 1.00 mg
- Aerosil: 2.58 mg
- Magnesum stearate: 1.00 mg

Ivabradine and Povidon VA 64 together with half of the Prosolv SMCC 90, magnesium stearate, Aerosil and the total amount of sodium bicarbonate were pre-mixed for 5 min. in the tumbling mixer (Turbula T10B) and compacted. Subsequently, the material was broken over a 1000 pm screen-type mill (Comil), the remaining excipients were added, followed by mixing for 5 min. in the tumbling mixer (Turbula T10B). The finished mixture was compressed on a rotary press (Riva Piccola) with 7 mm round biconvex punch. The tablets had a hardness of about 50-85 N.

The dissolution profile (conditions: 500 mL 0.1 nHCl pH 1.2, 37°C, 50 rpm baskets (USP app. I)) of the tablets of Example 6 is shown in Fig. 3.

As can be seen in comparison to Example 5 (direct compression), the direct compression of the same amount of active agents provide an improved dissolution profile compared to the dissolution profile of tablets obtained by compacting.

The pre-mixing of the active agent with the adhesion enhancer for 10 min. prior to further processing of the mixture provides an advantageous effect on the dissolution profile.

### Example 7: Stability of ivabradine adipate vs. ivabradine HCl

The stability of ivabradine adipate in comparison to ivabradine hydrochloride form I was investigated at different temperatures and humidities in open or closed containers for different storage times. The results are summarized in the following table.

**Table: Stabilty of Ivabradine adipate versus Ivabradine HCl, form I**

| Temp./humidity, Container, days | HCl Form I | Adipate |
|---|---|---|
| 25°C/60% closed, 33d | unchanged | |
| 25°C/60% closed, 57d | | unchanged |
| 25°C/60% open, 33d | β + unident. cryst. phase | |
| 25°C/60% open,57d | | unchanged |
| 30°C/65% closed, 33d | unchanged | |
| 30°C/65% closed, 57d | | unchanged |
| 30°C/65% open, 33d | β | |
| 30°C/65% open,57d | | unchanged |
| 40°C/75% closed, 33d | unchanged | |
| 40°C/75% closed, 57d | | unchanged |
| 40°C/75% open,33d | β | |
| 40°C/75% open,57d | | unchanged |
| Particle size D50 in µm | 19.10 | 18.35 |
| Particle size D90 in µm | 44.11 | 52.71 |

Ivabradine adipate according to the present invention is stable at various conditions. The ivabradine HCl form I undergoes phase transition into ivabradine HCl, form beta, or form d, in particular in open containers.

## Claims

1. Pharmaceutical composition containing ivabradine adipate as active substance wherein the active substance has an average particle size in the range of 0.5 µm to 250 µm, wherein the average particle size is determined by means of laser light diffraction.

2. Pharmaceutical composition according to claim 1 wherein the active substance has an average particle size in the range of 1 µm to 150 µm.

3. Pharmaceutical composition according to any one of the preceding claims wherein the composition additionally contains at least one adhesion enhancer, wherein the adhesion enhancer is a polymer selected from the group consisting of polysaccharides such as hydroxypropylmethylcellulose, carboxymethylcellulose, ethylcellulose, methylcellulose, hydroxyethylcellulose, ethylhydroxyethylcellulose, and hydroxypropylcellulose, microcrystalline cellulose, guar gum, alginic acid, alginates, polyvinylpyrrolidone, polyvinylacetates, polyvinyl alcohols, polymers of the acrylic acid and its salts, polyacrylamides, polymethacrylates, vinylpyrrolidone vinylacetate copolymers, polyalkylene glycoles such as poly(propylene glycol) or polyethylene glycol, co-blockpolymers of the polyethylene glycol, and mixtures of two or more of the mentioned polymers, or the polymer is selected from the group consisting of polyvinylpyrrolidone, copolymers of vinylpyrrolidone and vinylacetate, polyethylene glycols, hydroxypropylmethylcellulose, microcrystalline cellulose and mixtures of two or more of the mentioned polymers.

4. Pharmaceutical composition according to claim 3 wherein the weight ratio of ivabradine based on the free base to adhesion enhancer is in the range of 10:1 to 1:100, preferably in the range of 1:5 to 1:35.

5. Pharmaceutical composition according to any one of claims 3 or 4 wherein the adhesion enhancer has a volume average particle size in the range of 5 µm to 200 µm.

6. Pharmaceutical composition according to any one of the preceding claims wherein the composition is obtainable by direct compression methods in the absence of a solvent.

7. Pharmaceutical composition according to any one of the preceding claims wherein the composition is present as optionally film-coated tablet.

8. Method for the preparation of a pharmaceutical composition according to any one of claims 1 to 7 comprising the steps
a) obtaining ivabradine adipate as active substance wherein the active substance has an average particle size in the range of 0.5 µm to 250 µm; and
b) mixing the active substance with one or more pharmaceutically acceptable excipients.

9. Method according to claim 8 comprising the additional step of mixing with an adhesion enhancer as defined in claim 3.

10. Method according to any one of claims 8 or 9 comprising the additional step of direct compression in the absence of a solvent.

## Patentansprüche

1. Pharmazeutische Zusammensetzung, enthaltend Ivabradinadipat als aktive Substanz, wobei die aktive Substanz eine durchschnittliche Teilchengröße im Bereich von 0,5 µm bis 250 µm hat, wobei die durchschnittliche Teilchengröße mittels Laserlichtbeugung bestimmt wird.

2. Pharmazeutische Zusammensetzung nach Anspruch 1, wobei die aktive Substanz eine durchschnittliche Teilchengröße im Bereich von 1 µm bis 150 µm hat.

3. Pharmazeutische Zusammensetzung nach einem der vorstehenden Ansprüche, wobei die Zusammensetzung zusätzlich mindestens einen Haftverstärker enthält, wobei der Haftverstärker ein Polymer ist, ausgewählt aus der Gruppe, bestehend aus Polysacchariden wie Hydroxypropylmethylcellulose, Carboxymethylcellulose, Ethylcellulose, Methylcellulose, Hydroxyethylcellulose, Ethylhydroxyethylcellulose und Hydroxypropylcellulose, mikrokristalliner Cellulose, Guargummi, Alginsäure, Alginaten, Polyvinylpyrrolidon, Polyvinylacetaten, Polyvinylalkoholen, Polymeren der Acrylsäure und ihren Salzen, Polyacrylamiden, Polymethacrylaten, Vinylpyrrolidon-Vinylacetat-Copolymeren, Polyalkylenglycolen wie Poly(propylenglycol) oder Polyethylenglycol, Co-Blockpolymeren des Polyethylenglycols und Gemischen aus zwei oder mehr der genannten Polymere, oder das Polymer ausgewählt ist aus der Gruppe, bestehend aus Polyvinylpyrrolidon, Copolymeren von Vinylpyrrolidon und Vinylacetat, Polyethylenglycolen, Hydroxypropylmethylcellulose, mikrokristalliner Cellulose und Gemischen aus zwei oder mehr der genannten Polymere.

4. Pharmazeutische Zusammensetzung nach Anspruch 3, wobei das Gewichtsverhältnis von Ivabradin, basierend auf der freien Base, zum Haftverstärker im Bereich von 10 : 1 bis 1 : 100, bevorzugt im Bereich von 1 : 5 bis 1 : 35 liegt.

5. Pharmazeutische Zusammensetzung nach einem der Ansprüche 3 oder 4, wobei der Haftverstärker eine volumenmittlere Teilchengröße im Bereich von 5 µm bis 200 µm hat.

6. Pharmazeutische Zusammensetzung nach einem der vorstehenden Ansprüche, wobei die Zusammensetzung durch Direktkompressionsverfahren in Abwesenheit eines Lösungsmittels erhältlich ist.

7. Pharmazeutische Zusammensetzung nach einem der vorstehenden Ansprüche, wobei die Zusammensetzung als gegebenenfalls filmbeschichtete Tablette vorliegt.

8. Verfahren zur Herstellung einer pharmazeutischen Zusammensetzung nach einem der Ansprüche 1 bis 7, umfassend die Schritte
a) Erhalten von Ivabradinadipat als aktive Substanz, wobei die aktive Substanz eine durchschnittliche Teilchengröße im Bereich von 0,5 µm bis 250 µm hat; und
b) Mischen der aktiven Substanz mit einem oder mehreren pharmazeutisch akzeptablen Hilfsstoffen.

9. Verfahren nach Anspruch 8, umfassend den zusätzlichen Schritt des Mischens mit einem Haftverstärker, wie in Anspruch 3 definiert.

10. Verfahren nach einem der Ansprüche 8 oder 9, umfassend den zusätzlichen Schritt der direkten Kompression in Abwesenheit eines Lösungsmittels.

## Revendications

1. Composition pharmaceutique contenant l'adipate d'ivabradine comme substance active dans laquelle la substance active a une taille de particules moyenne dans l'intervalle de 0,5 µm à 250 µm, dans laquelle la taille moyenne de particule est déterminée au moyen de diffraction de lumière laser.

2. Composition pharmaceutique selon la revendication 1, dans laquelle la substance active a une taille de particules moyenne dans l'intervalle de 1 µm à 150 µm.

3. Composition pharmaceutique selon l'une quelconque des revendications précédentes dans laquelle la composition contient en plus au moins un augmentateur d'adhésion, dans laquelle l'augmentateur d'adhésion est un polymère sélectionné parmi le groupe constitué de polysaccharides tels que hydroxypropylméthylcellulose, carboxyméthylcellulose, éthylcellulose, méthylcellulose, hydroxyéthylcellulose, éthylhydroxyéthylcellulose, et hydroxypropylcellulose, cellulose microcristalline, gomme de guar, acide alginique, alginates, polyvinylpyrrolidone, polyvinylacétates, alcools polyvinyliques, polymères de l'acide acrylique et ses sels, polyacrylamides, polyméthacrylates, copolymères de vinylpyrrolidone-acétate de vinyle, polyalkylènes glycols tels que poly(propylène glycol) ou polyéthylène glycol, copolymères séquencés de polyéthylène glycol, et des mélanges de deux ou plus des polymères mentionnés, ou le polymère est sélectionné parmi le groupe constitué de polyvinylpyrrolidone, copolymères de vinylpyrrolidone et acétate de vinyle, polyéthylène glycols, hydroxypropylméthylcellulose, cellulose microcristalline et des mélanges de deux ou plus des polymères mentionnés.

4. Composition pharmaceutique selon la revendication 3 dans laquelle le rapport de poids de l'ivabradine basée sur la base libre à l'augmentateur d'adhésion est dans l'intervalle de 10:1 à 1:100, préférentiellement dans l'intervalle 1:5 à 1:35.

5. Composition pharmaceutique selon l'une quelconque des revendications 3 ou 4 dans laquelle l'augmentateur d'adhésion a une taille de particules moyenne en volume dans l'intervalle de 5 µm à 200 µm.

6. Composition pharmaceutique selon l'une quelconque des revendications précédentes dans laquelle la composition peut être obtenue par des méthodes de compression directe en absence d'un solvant.

7. Composition pharmaceutique selon l'une quelconque des revendications précédentes, dans laquelle la composition est présente comme comprimé éventuellement pelliculé.

8. Méthode pour la préparation d'une composition pharmaceutique selon l'une des revendications 1 à 7 comprenant les étapes
a) d'obtenir de l'adipate d'ivabradine comme substance active dans laquelle la substance active a une taille de particules moyenne dans l'intervalle de 0,5 µm à 250 µm ; et
b) de mélanger la substance active avec un ou plusieurs excipients pharmaceutiquement acceptables.

9. Méthode selon la revendication 8 comprenant l'étape additionnelle de mélange avec un augmentateur d'adhésion comme défini à la revendication 3.

10. Méthode selon l'une quelconque des revendications 8 ou 9 comprenant l'étape additionnelle de compression directe en absence d'un solvant.
